## Europäisches Patentamt

## European Patent Office

## Office européen des brevets

(19)

(11) Veröffentlichungsnummer: **0 016 962**
**B1**

(12) # EUROPÄISCHE PATENTSCHRIFT

(45) Veröffentlichungstag der Patentschrift:
**28.07.82**

(21) Anmeldenummer: **80100958.0**

(22) Anmeldetag: **26.02.80**

(51) Int. Cl.³: **C 12 Q 1/00**, C 12 Q 1/26, C 12 Q 1/54

(54) **Mittel zur Entfernung von Ascorbinsäure aus wässrigen Flüssigkeiten.**

(30) Priorität: **10.04.79 DE 2914487**

(43) Veröffentlichungstag der Anmeldung:
**15.10.80 Patentblatt 80/21**

(45) Bekanntmachung des Hinweises auf die Patenterteilung:
**28.07.82 Patentblatt 82/30**

(84) Benannte Vertragsstaaten:
**AT BE CH DE FR GB IT LU NL SE**

(56) Entgegenhaltungen:
**DE-A-2 630 043**
**FR-A-2 354 561**
**FR-A-2 363 107**
**US-A-4 076 502**

(73) Patentinhaber: **BOEHRINGER MANNHEIM GMBH,
Sandhofer Strasse 112-132 Postfach 31 01 20,
D-6800 Mannheim 31-Waldhof (DE)**

(72) Erfinder: **Deneke, Ulfert, Dr., Stettiner-Strasse 10C,
D-6942 Mörlenbach (DE)**
Erfinder: **Lange, Hans, Röntgenstrasse 14,
D-6840 Lampertheim (DE)**
Erfinder: **Rittersdorf, Walter, Dr., Kasseler Strasse 6,
D-6800 Mannheim-Waldhof (DE)**

(74) Vertreter: **Weickmann, Heinrich, Dipl.-Ing. et al,
Postfach 860 820 Möhlstrasse 22,
D-8000 München 86 (DE)**

## Mittel zur Entfernung von Ascorbinsäure aus wässrigen Flüssigkeiten

Die Erfindung betrifft ein Mittel zur Beseitigung von Ascorbinsäure aus wässrigen Lösungen.

In wässrigen Flüssigkeiten, insbesondere in wässrigen Lösungen physiologischer Herkunft sowie in Pflanzensäften und dergleichen, liegt häufig Ascorbinsäure vor. Ascorbinsäure ist bekanntlich ein starkes Reduktionsmittel, welches unter Umständen stören kann, insbesondere wenn andere Inhaltsstoffe der wässrigen Flüssigkeiten analytisch bestimmt werden sollen. So kann Ascorbinsäure bei der Analyse von Urin auf pathologische Bestandteile beispielsweise sehr störend in Erscheinung treten. Viele Bestimmungsmethoden, insbesondere aber Schnelldiagnostika zur Bestimmung von Glucose, Blut, Nitrit, Urobilinogen und Bilirubin werden von Ascorbinsäure mehr oder weniger stark im Sinne einer Vortäuschung niedrigerer Konzentrationen gestört. Bei der Fehling'schen Probe auf Glucose wird deren Vorhandensein dagegen durch Ascorbinsäure vorgetäuscht, so dass falsche positive bzw. zu hohe Werte gemessen werden.

Da das Vorkommen von Ascorbinsäure im Urin, bedingt durch die reichliche alimentäre Aufnahme, sehr häufig ist, hat man schon verschiedentlich versucht, die Ascorbinsäure vor der Analyse der gesuchten Bestandteile aus dem Harn zu entfernen. Folgende Verfahren sind bereits bekannt:

Oxidation mit Jodlösung und Entfernung des überschüssigen Jods mit Thiosulfatlösung,

Oxidation mit Mangandioxid und Abfiltrieren des nicht verbrauchten Oxidationsmittels,

Oxidation mit alkalischem $H_2O_2$,

Behandlung des Harns mit einem Anionenaustauscher.

Alle diese Verfahren erfordern eine umständliche Behandlung des Harns, so dass sie sich in der Praxis nicht durchsetzen konnten. Ausserdem werden dabei unter Umständen nachzuweisende Stoffe zerstört oder vorgetäuscht.

Bekannt sind auch Testpapiere, bei denen der Urin zuerst durch eine Zone mit oxidierendem Material bzw. Anionenaustauscher (DE-B-Nr. 1598008) chromatographiert werden muss, wobei die Ascorbinsäure abgetrennt wird, und dann beim Weiterlaufen auf der eigentlichen Reagentszone ungestört reagiert. Testpapiere dieser Art sind für Glucose und Galactose handelsüblich. Sie weisen jedoch einen komplizierten Aufbau auf. Ausserdem wird durch die erforderliche Chromatographierzeit der Zeitaufwand erheblich gesteigert.

Gemäss einem älteren Vorschlag der Anmelderin ist es bereits bekannt, Ascorbinsäure durch Zusatz von Ascorbatoxidase zu entfernen (DE-B-Nr. 2625834). Dieses Verfahren ist zwar brauchbar, benötigt jedoch zur Beseitigung der Störung, insbesondere wenn mit dem Vorhandensein grösserer Mengen Ascorbinsäure gerechnet werden muss, eine grosse Menge an Ascorbatoxidase. Für viele Fälle ist daher der Einsatz dieses Verfahrens bzw. von nach diesem Verfahren wirksamen Testpapieren durch den benötigten hohen Einsatz an Ascorbatoxidase unwirtschaftlich. Ein weiterer Nachteil dieses Verfahrens besteht darin, dass die Lösung, in der die Ascorbinsäure entfernt werden muss, kräftig geschüttelt werden muss, damit der für die Reaktion benötigte Sauerstoff in Lösung geht.

Der Erfindung liegt daher die Aufgabe zugrunde, ein Mittel zur Entfernung von Ascorbinsäure in wässrigen Flüssigkeiten zu schaffen, welches die oben beschriebenen Nachteile der bekannten Verfahren und Mittel vermeidet und sich rasch, zuverlässig und ohne besondere Hilfsmittel verwenden lässt und wirtschaftlich tragbar ist.

Gelöst wird diese Aufgabe durch ein Mittel zur Beseitigung von Ascorbinsäure aus wässrigen Flüssigkeiten, auf Basis eines festen, perösen, saugfähigen, mit Ascorbatoxidase imprägnierten Trägermaterials, welches dadurch gekennzeichnet ist, dass das Trägermaterial zusätzlich mit Katalase und, von den Enzymen räumlich getrennt, mit $H_2O_2$, sowie gegebenenfalls mit Stabilisatoren oder/und Puffersubstanz imprägniert und an einem weiteren kompakten Trägermaterial befestigt ist.

Aus der älteren, nicht vorveröffentlichten europäischen Anmeldung EP-A-Nr. 80100597 ist ein Proberöhrchen insbesondere für Urin-Proben zu entnehmen, welches an einem Teil seiner Innenwandung eine Schicht aufweist, die Katalase und Ascorbinsäureoxidase enthält und eine sauerstoffabgebende Substanz wie ein Peroxid als Trockensubstanz in dem Röhrchen enthalten ist. Das erfindungsgemässe Mittel ist dort jedoch nicht beschrieben.

Aus der US-A-Nr. 4076502 sind Teststreifen bekannt, bei denen die Reaktion in einem saugfähigen, mit Reagenzien imprägnierten Trägermaterial stattfindet. Ein Mittel, welches Ascorbatoxidase, Katalase und $H_2O_2$ enthält, lässt sich daraus nicht herleiten.

Das erfindungsgemässe Mittel ermöglicht eine rasche und sichere Entfernung von Ascorbinsäure aus wässrigen Flüssigkeiten, wie Urin. Dies ist überraschend, weil hierbei eine Sauerstoffentwicklung in der Lösung auftritt, welche die Löslichkeit des Sauerstoffs in der wässrigen Flüssigkeit weit übersteigt, so dass zu erwarten war, dass gebildeter Sauerstoff grösstenteils durch Gasentwicklung verlorengehen würde. Auch war ein negativer Einfluss des $H_2O_2$ auf die Gleichgewichtslage der Reaktion zu befürchten gewesen, nachdem bei vielen Untersuchungen $H_2O_2$ als Endprodukt der Oxidation von Ascorbinsäure durch Ascorbatoxidase gefunden worden ist. Unerwarteterweise können jedoch erfindungsgemäss mit der gleichen Ascorbatoxidasemenge weit grössere Ascorbinsäuremengen zerstört werden als in Abwesenheit von $H_2O_2$ und Katalase bzw. die zur Entfernung einer bestimmten Ascorbonsäuremenge erforderliche Ascorbatoxidasemenge kann wesentlich verringert werden.

Besonders überraschend ist ausserdem, dass dieses Mittel selbst dann anwendbar ist, wenn anschliessend eine Reaktion durchgeführt wird, bei der gebildetes $H_2O_2$ den Messparameter darstellt. Dies ist beispielsweise der Fall bei der Glucosebestimmung mit Glucoseoxidase unter Messung von gebildetem $H_2O_2$ mit 4-Aminophenazon und Phenol. Katalase wird im Rahmen des erfindungsgemässen Mittels zweckmässig in Form eines handelsüblichen Präparats verwendet. $H_2O_2$ kann als solches verwendet werden. Vorzugsweise wird es jedoch in Form eines festen Addukts, d.h. einer Anlagerungsverbindung von $H_2O_2$ an eine andere Substanz, eingesetzt. Bevorzugte Beispiele hierfür sind Perborate, Harnstoffperhydrat und Mannitperhydrat.

Als Trägermaterial eignen sich inerte Substanzen, welche mit Ascorbatoxidase, Katalase und $H_2O_2$ bzw. den festen $H_2O_2$-Addukten imprägniert werden können, insbesondere die zur Herstellung von Teststreifen bekannten Trägermaterialien. Typische Beispiele hierfür sind saugfähige Papiere und papierartige Substanzen auf Basis natürlicher oder synthetischer bzw. künstlicher Fasern, Vliese, offenzellige Schaumstoffe, poröses Glas, aus anorganischen Stoffen, wie Glas, bestehende Fasermatten und dergleichen.

Das Trägermaterial enthält die Reagenzien entweder einzeln, d.h. in voneinander räumlich getrennten Abschnitten, oder ein Gemisch der Enzyme getrennt vom $H_2O_2$. Letztere Ausführungsform wird bevorzugt, da es sich überraschenderweise gezeigt hat, dass die beiden Enzyme Ascorbatoxidase und Katalase eine sich gegenseitig stabilisierende Wirksamkeit aufweisen und dadurch die Lagerfähigkeit des imprägnierten Trägermaterials wesentlich verbessert wird. Es ist daher möglich, die beiden Enzyme ohne zusätzliche Stabilisatoren gemeinsam auf dem Trägermaterial vorzusehen. Bevorzugt werden jedoch ausserdem übliche Enzymstabilisatoren oder/und Puffersubstanzen, die einen für die Stabilität dieser Enzyme geeigneten pH-Wert liefern, dem Trägermaterial ebenfalls einverleibt.

Das $H_2O_2$, welches vorzugsweise in Form eines festen Addukts, wie oben erwähnt, im Trägermaterial vorliegt, wird zweckmässig ebenfalls stabilisiert, wobei die üblichen $H_2O_2$-Stabilisatoren zur Anwendung kommen können. Sehr gute Stabilisierungsergebnisse wurden mit Pektin, Polyvinylpyrrolidon oder Dextran bzw. Mischungen derselben erzielt.

Die Herstellung des imprägnierten Trägermaterials erfolgt einfach, indem das Material mit einer Lösung des aufzubringenden Stoffs oder Stoffgemisches imprägniert und dann getrocknet wird. Beispielsweise werden geeignete Papiere mit einer Ascorbatoxidase, Katalase, Stabilisatoren und Puffermaterial enthaltenden Lösungen imprägniert und getrocknet, während ein anderes Trägermaterial mit der ebenfalls vorzugsweise Stabilisierungsmittel enthaltenden Lösung des $H_2O_2$-Addukts getränkt und in gleicher Weise getrocknet wird. Zweckmässig bemessene Stücke dieser so hergestellten imprägnierten Trägermaterialien können dann an einem geeigneten kompakten Basisträgermaterial befestigt werden, beispielsweise durch Aufkleben. Man erhält so ein Mittel der oben bezeichneten Art, welches ein kompaktes Trägermaterial aufweist, an welchem wenigstens ein poröses saugfähiges Trägermaterial befestigt ist, das mit einem oder mehreren der oben genannten Wirkstoffe imprägniert ist. Das kompakte Trägermaterial weist dabei zweckmässig Band- oder Stabform auf. Man kann dann z.B. ein derartiges Stäbchen in die Ascorbinsäure enthaltende Lösung eintauchen, so dass die Reagenzien von dem porösen Trägermaterial abgelöst werden und die Reagenzien durch einfaches Umrühren, wobei das kompakte Trägermaterial als Handgriff dient, mischen und den Ablauf der Reaktion beschleunigen. Derartige, zum Umrühren geeignete Stäbchen oder Bänder werden häufig auch als Plümper bezeichnet. Das kompakte Trägermaterial kann aus beliebigem inertem Material bestehen, zweckmässig verwendet man Glas oder Kunststoff. Beispielsweise kann man ein Kunststoffstäbchen als kompaktes Trägermaterial mit zwei Papierstreifen bekleben, von denen einer mit Ascorbatoxidase und Katalase, der andere mit $H_2O_2$ imprägniert ist. Der so erhaltene Plümper wird dann in die wässrige Flüssigkeit, beispielsweise den Urin, eingetaucht und kurz umgerührt, wobei sich die Reagenzien ablösen, gut durchmischt werden und die Ascorbinsäure in kurzer Zeit vollständig zerstört wird.

Wenn das erfindungsgemässe Mittel eine Puffersubstanz enthält, so wird zweckmässig eine solche verwendet, welche einen pH-Wert zwischen 5 und 8,5 aufrechterhalten kann. Besonders geeignet als Puffersubstanz erwies sich dabei Triscitratpuffer oder/zu Zitronensäure. Letztere eignet sich besonders dann, wenn ein festes $H_2O_2$-Addukt, welches stark alkalisch reagiert, vorliegt.

Die Menge an Reagenzien im erfindungsgemässen Mittel wird in erster Linie durch den vorgesehenen Verwendungszweck bestimmt. Soll es als Plümper zur Entfernung von Ascorbinsäure in Urinproben verwendet werden, so hat es sich als zweckmässig erwiesen, wenn die Ascorbatoxidasemenge wenigstens 30 U, vorzugsweise 35 bis 40 U beträgt. Für andere wässrige Flüssigkeiten, wie z.B. Fruchtsäfte, werden die Mengen je nach dem zu erwarteten Ascorbinsäuregehalt gewählt.

In der beigefügten Zeichnung stellen dar:

Fig. 1 ein erfindungsgemässes Mittel zur Beseitigung von Ascorbinsäure in wässrigen Flüssigkeiten in der Aufsicht,

Fig. 2 das Mittel von Fig. 1 in Seitenansicht.

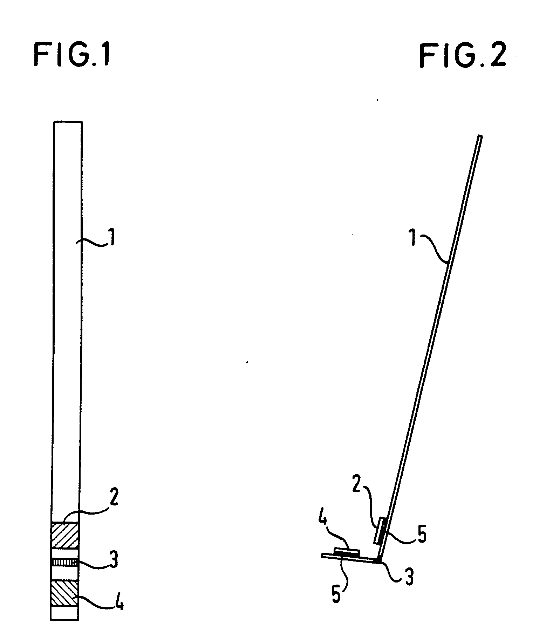

Die in den Fig. 1 und 2 dargestellte Ausführungsform des erfindungsgemässen Mittels ist als Plümper aufgebaut. Ein Kunststoffstreifen 1, der das kompakte Trägermaterial darstellt, trägt nahe seinem unteren Ende einen mit 35 $\mu$mol $H_2O_2$ imprägnierten Papierstreifen 2 und einen mit 36 U Ascorbatoxidase und 10 000 U Katalase imprägnierten Papierstreifen 4. Zwischen den beiden Papierstreifen 2 und 4 befindet sich eine farblich markierte Sollknickstelle 3. Durch Umbiegen an der Sollknickstelle erhält der Kunststoffstreifen die Form eines gut rührfähigen Spatels. Die beiden porösen saugfähigen Träger 2 und 4 sind mittels Kleber 5 am Kunststoffstreifen 1 befestigt.

Die Erfindung eignet sich für Körperflüssigkeiten, wie Urin, Serum und Plasma sowie für andere, Ascorbat enthaltende wässrige Flüssigkeiten, wie z.B. Säfte von Obst und anderen Pflanzen.

Die folgenden Beispiele erläutern die Erfindung weiter.

*Beispiel 1 (Vergleich)*

*Entfernung grosser Mengen Ascorbat aus Urin mit und ohne Schütteln*

Ein Urin wird mit Ascorbinsäure auf einen Gehalt von 20 mg Ascorbat pro 100 ml aufgestockt. Je 10 ml dieser Probe werden mit 10 U Ascorbatoxidase versetzt. Die eine Probe wird leicht durchmischt, nach 10minütigem Stehen wird der Gehalt an Ascorbat bestimmt. Es finden sich noch 67% des zugesetzten Ascorbats. Die andere Probe wird 10min stark geschüttelt. In ihr ist nach dieser Zeit kein Ascorbat mehr nachweisbar. In zwei weiteren Proben, denen keine Ascorbatoxidase zugesetzt wird, findet sich mit und ohne Schütteln nach 10 min noch fast vollständig die zugesetzte Ascorbinsäure.

*Beispiel 2*

*Herstellung eines Ascorbatoxidasespatels*

a) Herstellung der Imprägnierlösung 1:
Ascorbatoxidase 83 000 U (10 000 bis 160 000 U);
Katalase 83 Mio U (40 bis 160 Mio U);
Mannit 15 g;
Triscitrat je 0,2 mol/l, pH 7,6, werden in Wasser gelöst und die Lösung mit Wasser aufgefüllt auf 100 ml. Mit dieser Lösung wird ein geeignetes Papier getränkt. Nach dem Trocknen schneidet man das Papier in 6 mm breite Streifen, die mit einem Kunststoffträger verklebt werden. Nach dem erneuten Schneiden enthalten die entstehenden 6×6 mm Bezirke je 36 U Ascorbatoxidase und 10 000 U (5000 bis 15 000 U) Katalase.

b) Herstellung von Imprägnierlösung 2:
Lösung A: Pektin 5 g werden in Wasser bidest. 100 ml gelöst.
Lösung B: Kollidon 25 33,4 g, Wasser 66,6 ml werden gemischt.

Die Lösungen A und B werden gemischt und darin Harnstoffperhydrat 40 g und Kollidon 25 50 g aufgelöst. Mit dieser Lösung wird ein geeignetes Papier getränkt und in 6 mm breite Streifen geschnitten. Diese Streifen werden auf dem gleichen Kunststoffträger, der bereits das ascorbatoxidasehaltige Papier enthält, geklebt. Nach dem Schneiden enthält das entstehende 6×6 mm grosse Testfeld 60 (50 bis 100) μmol Harnstoffperhydrat.

Der Kunststoffträger weist zwischen dem Ascorbatoxidasetestbezirk und dem Harnstoffperhydrattestbezirk eine Sollknickstelle auf, die vor Gebrauch umgebogen wird, um einen rührfähigen Spatel zu erzeugen. Mit diesem so gebildeten Spatel werden 10 ml eines Harns, der 50 mg Ascorbinsäure/100 ml enthält, kurz gerührt und der Spatel 10 min in dem Urin belassen. Nach 10 min wird der Spatel entnommen und der Restgehalt an Ascorbinsäure bestimmt. Es ist kein Ascorbat mehr feststellbar. Der so entstörte Harn ist geeignet, mittels Teststäbchen auf Nitrit, auf den pH-Wert, auf Eiweiss, auf Glucose, auf Ketonkörper, auf Bilirubin, auf Urobilinogen und auf Blut störungsfrei überprüft zu werden.

*Beispiel 3*

*Entfernung von Ascorbat aus Orangensaft*

Mit dem in Beispiel 2 beschriebenen Träger können 10 ml eines Orangensafts mit einem Gehalt von 350 mg Ascorbat/l in gleicher Weise wie in Beispiel 2 beschrieben behandelt werden. Auch hier ist danach kein Ascorbat mehr festzustellen.

*Beispiel 4*

15 ml Urin, enthaltend 150 bis 250 mg/100 ml Glucose und 50 mg/100 ml Ascorbat wurden mit Teststreifen gemäss DE-AS Nr. 2625834, Beispiel 8, auf Glucose geprüft. Die Reaktion ist negativ, da Ascorbat zur vollständigen Unterdrückung führt. Anschliessend wird mit dem Spatel gemäss Beispiel 2 geführt, wobei die Ascorbinsäure entfernt wird. Bei erneuter Prüfung auf Glucose mit dem oben erwähnten Teststreifen erhält man ein entsprechendes Signal. Das ist überraschend, da man erwarten sollte, dass die Katalase das im Glucosenachweis gebildete $H_2O_2$ zerstört.

*Beispiel 5*

Ascorbat stört photometrische Glucosebestimmungen, die auf der Reaktion mit GOD, POD, Phenol und 4-Aminophenazon beruhen. Der Spatel wird daher auch zur Entfernung von Ascorbat aus Proben für die Glucosebestimmung mit der GOD-PAP-Methode eingesetzt. Dazu werden 15 ml Urin mit dem Spatel gemäss Beispiel 2 behandelt und auf diese Weise von bis zu 50 mg Ascorbat befreit. Anschliessend werden 0,02 ml dieses Urins in 3 ml folgender Reagensmischung gegeben:

| Phosphat-Puffer | 0,12 mmol/l, pH 7,0 |
|---|---|
| POD | 1,5 U/ml |
| GOD | 19 U/ml |
| 4-Aminophenazon | 0,92 mmol/l |
| Phenol | 11 mmol/l |

Vor Probenzugabe wird $E_1$ bestimmt, nach Probenzugabe wird 40 min inkubiert und $E_2$ bestimmt. In gleicher Weise wird ein Glucosestandard gemessen und daraus der Glucosegehalt des Harns bestimmt. Durch die Behandlung des Probenurins mit Katalase können bis zu 1000 U/ml Katalase in den obigen photometrischen Test eingesetzt werden, ohne dass eine Störung des Tests auftritt.

**Patentansprüche**

1. Mittel zur Beseitigung von Ascorbinsäure in wässrigen Flüssigkeiten, auf Basis eines festen, porösen, saugfähigen, mit Ascorbatoxidase imprägnierten Trägermaterials, dadurch gekennzeichnet, dass das Trägermaterial zusätzlich mit Katalase und, von den Enzymen räumlich getrennt, mit H₂O₂, sowie gegebenenfalls mit Stabilisatoren oder/und Puffersubstanz imprägniert und an einem weiteren kompakten Trägermaterial befestigt ist.

2. Mittel nach Anspruch 1, dadurch gekennzeichnet, dass das poröse Trägermaterial Ascorbatoxidase und Katalase gemischt enthält.

3. Mittel nach Anspruch 1 oder 2, dadurch gekennzeichnet, dass das H₂O₂ als festes Addukt vorliegt.

4. Mittel nach einem der Ansprüche 1 bis 3, dadurch gekennzeichnet, dass es als Stabilisatoren für H₂O₂ Pektin, Polyvinylpyrrolidon oder Dextran oder Mischung derselben enthält.

5. Mittel nach Anspruch 1, dadurch gekennzeichnet, dass das kompakte Trägermaterial Band- oder Stabform aufweist.

6. Mittel nach Anspruch 1 oder 5, dadurch gekennzeichnet, dass das kompakte Trägermaterial aus Glas oder Kunststoff besteht.

7. Mittel nach einem der Ansprüche 1 bis 6, dadurch gekennzeichnet, dass es eine bei einem pH-Wert zwischen 5 und 8,5 wirksame Puffersubstanz enthält.

8. Mittel nach Anspruch 7, dadurch gekennzeichnet, dass es Triscitratpuffer oder/und Zitronensäure enthält.

9. Mittel nach einem der Ansprüche 1 bis 8, dadurch gekennzeichnet, dass es wenigstens 30 U Ascorbatoxidase enthält.

## Claims

1. Agent for the removal of ascorbic acid from aqueous liquids, based upon a solid, porous, absorbent material impregnated with ascorbate oxidase, characterised in that the carrier material is additionally impregnated with catalase and, spatially separated from the enzymes, with H₂O₂, as well as optionally with stabilisers and/or buffer substances and is fixed to a further compact carrier material.

2. Agent according to claim 1, characterised in that the porous carrier material contains ascorbate oxidase and catalase mixed.

3. Agent according to claim 1 or 2, characterised in that the H₂O₂ is present as a solid adduct.

4. Agent according to one of claims 1 to 3, characterised in that, as stabilisers for H₂O₂, it contains pectin, polyvinylpyrrolidone or dextran or mixtures thereof.

5. Agent according to claim 1, characterised in that the compact carrier material has a strip or rod shape.

6. Agent according to claim 1 or 5, characterised in that the compact carrier material consists of glass or synthetic resin.

7. Agent according to one of claims 1 to 6, characterised in that it contains a buffer substance effective at a pH value between 5 and 8.5.

8. Agent according to claim 7, characterised in that it contains triscitrate buffer and/or citric acid.

9. Agent according to one of claims 1 to 8, characterised in that it contains at least 30 U of ascorbate oxidase.

## Revendications

1. Dispositif pour éliminer l'acide ascorbique dans les liquides aqueux, à base d'une matière-support solide, poreuse, absorbante, imprégnée d'ascorbateoxydase, caractérisé en ce que la matière-support est en outre imprégnée de catalase et séparée, dans l'espace des enzymes, de H₂O₂, ainsi qu'éventuellement de stabilisants ou/et de substance-tampon et est fixée à une autre matière support compacte.

2. Dispositif selon la revendication 1, caractérisé en ce que la matière-support poreuse contient de l'ascorbateoxydase et de la catalase mélangées.

3. Dispositif selon l'une des revendications 1 ou 2, caractérisé en ce que le H₂O₂ se présente sous forme d'adduct solide.

4. Dispositif selon l'une des revendications 1 à 3, caractérisé en ce qu'il contient en tant que stabilisants pour H₂O₂, de la pectine, de la polyvinylpyrrolidone ou du dextrane ou des mélanges de ceux-ci.

5. Dispositif selon la revendication 1, caractérisé en ce que la matière-support compacte présente une forme de ruban ou de baguette.

6. Dispositif selon la revendication 1 ou 5, caractérisé en ce que la matière-support compacte est constituée par du verre ou de la matière plastique.

7. Dispositif selon l'une des revendications 1 à 6, caractérisé en ce qu'il contient une substance-tampon active à une valeur de pH entre 5 et 8,5.

8. Dispositif selon la revendication 7, caractérisé en ce qu'il contient du tampon au triscitrate ou/et de l'acide citrique.

9. Dispositif selon l'une des revendications 1 à 8, caractérisé en ce qu'il contient au moins 30 U d'ascorbateoxydase.

# FIG.1

# FIG.2